# EUROPEAN PATENT APPLICATION

(11) **EP 3 919 061 A1**
(43) Date of publication of application: **08.12.2021**
(21) Application number: 20748654.9
(22) Date of filing: 29.01.2020
(51) Int. Cl.: A61K 31/664, A61K 31/665, A61K 9/127, A61K 9/107, A61P 31/04

(54) **USE OF P1P DERIVATIVE AS THERAPEUTIC AGENT FOR SEPSIS**

(30) Priority: 30.01.2019 KR 20190011742
(71) Applicant: Axceso Biopharma Co., Ltd., Gyeonggi-do 14056 (KR)
(72) Inventor: LEE, Jeong Min, Anyang-si Gyeonggi-do 14005 (KR); KIM, Su Jin, Bucheon-si Gyeonggi-do 14482 (KR); HAN, Won Kyo, Gwangju-si Gyeonggi-do 12771 (KR); PARK, Young Jun, Yongin-si Gyeonggi-do 16995 (KR); CHOI, Myeong Jun, Seoul 06286 (KR)
(74) Representative: V.O.
(86) International application number: PCT/KR2020/001377
(87) International publication number: WO 2020/159233

(57) **Abstract**

The present disclosure provides a pharmaceutical composition for preventing or treating sepsis or septic shock comprising P1P or a derivative thereof. The present composition improves the survival rate of mice with sepsis induced by LPS or CLP treatment, as well as reduces the secretion of cytokines. Further, it helps to enhance the proliferation and function of vascular endothelial cells and increases the expression of SIRT1 protein which decreases in a sepsis patient. Accordingly, it can be effectively used in the treatment of sepsis.

## Description

### Technical Field

The present disclosure relates to a composition for preventing or treating sepsis or septic shock comprising phytosphingosine-1-phosphate (P1P) or a derivative thereof as an active ingredient.

### Background Art

Sepsis is an inflammatory reaction induced by excessive activation of the body's immune system by infection with pathogenic microorganisms. In severe cases, it leads to shock and death in the patient.

Specifically, sepsis is a systemic inflammatory reaction syndrome caused by infection and mainly occurs acutely in infants, the elderly, or surgical patients with weak immunity. Sepsis is mostly accompanied by a systemic inflammatory response, and systemic inflammatory response can be caused by various causes. A systemic inflammatory response caused by infection with pathogenic microorganisms in the body is called sepsis. Sepsis is a disease that accounts for the majority of deaths in critically ill patients, and is a common infectious disease accounting for about 25-30 % of hospitalized patients. Sepsis is a dangerous condition with a mortality rate of 30-60 %, but there is no effective treatment.

Treatment of sepsis starts with the identification of the infection, and the treatment consists of various methods such as infection control, hemodynamic support, host immunomodulation, and metabolic/endocrine support. Above all, the most important thing in sepsis treatment is to diagnose sepsis as soon as possible.

Furthermore, selection of antibiotics is very important in the treatment of sepsis, and strong and broad-spectrum antibiotics are primarily used in a state where the causative pathogen is unknown. In patients with severe sepsis or septic shock, an appropriate antibiotic should be administered intravenously within 1 hour after diagnosis to reduce mortality. The timing of the use of antibiotics and the selection of drugs are also very important. It is known to be effective to use one or two or more antibiotics effective against the likely causative pathogens in combination. In addition to antibiotic therapy, fluid therapy, vasopressor therapy, use of cardiac stimulants and blood products, insulin therapy, and corticosteroids are also used.

Various attempts are being made to develop a therapeutic agent in the absence of an effective therapeutic agent for sepsis. In particular, many attempts have been made as a method of inhibiting inflammation, but various anti-inflammatory drugs often fail in clinical practice, so a new treatment strategy is needed rather than a method to inhibit inflammation. Among them, drugs that are widely used for the treatment of hyperlipidemia (Simvastatin, Cerivastatin, Fluvastatin, Ulinastatin) are widely used in sepsis research. If these drugs are pretreated before LPS (Lipopolysaccharide) treatment, the effect of improving the survival rate by 40-60 % is shown, but when LPS is first treated, the effect is lowered.

As another method for the treatment of sepsis, various methods using stem cells have been tried. Methods using adipose stem cells, mesenchymal stem cells, bone marrow-derived stem cells, or umbilical cord blood stem cells have been tried, but they do not have much effect. In the sepsis model treated with LPS, the method using stem cells improved the survival rate by 30-40 % compared to the LPS-treated group.

Recently, it was found that the activity and synthesis of SIRT1 protein and the content of sphingosine-1-phosphate (S1P) were decreased in sepsis patients compared to the normal group (Critical care, 2015, 19, 372; Free radical Biology and Medicine, 2017, 113, 291-303). S1P content tends to decrease sharply in sepsis patients, so an attempt is being made to treat sepsis by using an S1P agonist to increase the S1P content or by using an inhibitor that inhibits an enzyme that degrades S1P *(*Circulation research, 2008, 103, 1164-1172; Biochimica et Biophysica Acta, 2014, 1841, 1403-1412; J Pharmacol Exp Ther., 2015, 352, 61-66), and attempts are being made to increase SIRT1 protein content and activity decreased in sepsis patients. It is reported that increasing SIRT1 activity not only inhibits inflammation but also reduces vascular endothelial cell permeability, thereby improving sepsis. The best way to increase the content of S1P is to overproduce the gene that synthesizes S1P or knock down or knock out the enzyme that degrades S1P, but this method is inconvenient due to genetic manipulation. Also, the method using the S1P agonist has insignificant therapeutic effect. In particular, FTY720 (fingolimod), known as an S1P agonist, was also reported to have very low therapeutic effect. Stem cell monotherapy and stem cell and FTY720 combination therapy show about 10 % therapeutic effect. There is a method of administering S1P directly, but it is very expensive and has a problem with a low therapeutic effect because of its high degradation rate in the blood *(*Acta Pharmacologica Sinica, 2018, 0, 1-12; Free radical biology and medicine, 2017, 113, 291-303; Biochimica et Biophysica Acta, 2018, 1864(3), 784-792). Therefore, there is an urgent need to develop a new, safe and highly effective treatment for sepsis.

### Technical Problem

An object of the present disclosure is to provide a novel therapeutic agent for sepsis that is safe and has excellent therapeutic effects.

### Technical Solution

In one aspect, the present disclosure provides a pharmaceutical composition for preventing or treating sepsis or septic shock comprising phytosphingosine-1-phosphate (P1P), cP1P (O-cyclic P1P) or a pharmaceutically acceptable salt thereof.

In one embodiment of the present disclosure, the sepsis or septic shock may be induced by Gram-negative bacteria.

In one embodiment of the present disclosure, the sepsis or septic shock may be induced by Gram-negative bacteria-derived lipopolysaccharide (LPS).

In one embodiment of the present disclosure, the pharmaceutical composition may reduce the secretion of inflammatory cytokines, particularly TNF-α.

In one embodiment of the present disclosure, the pharmaceutical composition may promote proliferation of vascular endothelial cells.

In one embodiment of the present disclosure, the pharmaceutical composition may be a nanoparticle formulation selected from the group consisting of liposomes, nanoemulsions and micelles, and in particular may be a liposome formulation.

In another aspect, the present disclosure provides a method for preventing or treating sepsis or septic shock comprising administering to a subject in need thereof an effective amount of phytosphingosine-1-phosphate (P1P), cP1P (O-cyclic P1P) or a pharmaceutically acceptable salt thereof.

In another aspect, the present disclosure provides a use of phytosphingosine-1-phosphate (P1P), cP1P (O-cyclic P1P) or a pharmaceutically acceptable salt thereof for preventing or treating sepsis or septic shock.

### Advantageous Effects

The composition comprising P1P or a derivative thereof according to the present disclosure can effectively treat sepsis. Specifically, P1P or a derivative thereof according to the present disclosure shows an effect of improving the reduction in survival rate by LPS or CLP treatment in a sepsis model induced by LPS injection or CLP surgery, which is a representative method for inducing sepsis. These drugs are confirmed to ameliorate sepsis by inhibiting oxidative stress as well as inflammation. In addition, it is confirmed that P1P or derivatives thereof not only help to improve the proliferation and function of vascular endothelial cells, which are important for sepsis treatment, but also increase the amount of SIRT1 protein that is reduced in sepsis, so these drugs can effectively treat sepsis at various stages.

Further, it is confirmed that when P1P or a derivative thereof according to the present disclosure is administered in the form of liposome nanoparticles, the solubility is improved, and the therapeutic effect for sepsis is also increased, and thus sepsis can be effectively treated with a liposome formulation of P1P or a derivative thereof.

### Brief Description of Figures

Fig. 1 is a graph comparing the survival rates of Balb/c mice according to the LPS concentration.
Fig. 2a is a graph showing the effect of P1P on the survival rate when the P1P drug is administered 5 minutes after LPS (150 µg/mouse) treatment.
Fig. 2b is a graph showing the effect of P1P on the survival rate when the P1P drug is administered 60 minutes after LPS (150 µg/mouse) treatment.
Fig. 3a is a graph showing the effect of P1P on the survival rate when P1P drug of 40 µg/mouse is administered 60 minutes after LPS (125 µg/mouse) treatment.
Fig. 3b is a graph showing the effect of P1P on the survival rate according to the concentration of P1P drug when P1P is administered 60 minutes after LPS (125 µg/mouse) treatment.
Fig. 4a is a graph showing the effect of cP1P on the survival rate when the cP1P drug of 40 µg/mouse is administered 5 minutes after LPS (150 µg/mouse) treatment.
Fig. 4b is a graph showing the effect of cP1P on the survival rate when the cP1P drug of 40 µg/mouse is administered 60 minutes after LPS (125 µg/mouse) treatment.
Fig. 5 is a graph showing the effect of P1P and its derivative drugs on cytokine (TNF-α) secretion after LPS treatment in macrophages compared to the effect of S1P. Since sepsis leads to excessive cytokine storm following the initial infection, the excellent inhibition of TNF-α production by P1P and derivatives thereof according to the present disclosure, in particular, superior inhibition of cytokine compared to S1P, proves the excellent therapeutic effect of the pharmaceutical composition according to the present disclosure.
Fig. 6 is a graph showing the effects of P1P and cP1P drugs on the proliferation of human vascular endothelial cells compared to the effect of S1P. These results indicate that the composition according to the present disclosure is effective in treating sepsis.
Fig. 7 is a graph showing the effects of P1P and cP1P drugs on the survival of human vascular endothelial cells due to oxidative stress compared to the effect of S1P. The results show that the composition according to the present disclosure is effective in treating sepsis compared to S1P.
Fig. 8 is a graph showing the effect of P1P and cP1P drugs on the expression of SIRT1 protein in macrophages.
Fig. 9 is a graph showing the effect of cP1P on the survival rate when cP1P-liposome (cP1P concentration of 20 µg/mouse) was administered 6 hours and 18 hours after CLP treatment.

### Best Mode

The present disclosure is based on finding that phytosphingosine-1-phosphate or a derivative thereof is effective in treating sepsis.

Accordingly, in one embodiment, the present disclosure relates to a pharmaceutical composition for preventing or treating sepsis or septic shock comprising one or more compounds selected from the group consisting of phytosphingosine-1-phosphate (P1P), cP1P (O-cyclic P1P), and pharmaceutically acceptable salts thereof.

Phytosphingosine-1-phosphate (P1P) (Chemical Formula I) and cP1P (O-cyclic P1P) (Chemical Formula II) according to the present disclosure are derivatives of sphingosine-1-phosphate (S1P). These compounds were developed for various uses by the present inventors and have been patented and described in Korean Patent Nos. 10-1003532, 10-1340556 (new substance and its use for hair loss treatment) and No. 10-1514970 (composition for the treatment or prevention of atopic dermatitis or skin wounds). They are represented by the following Chemical Formulas I and II.

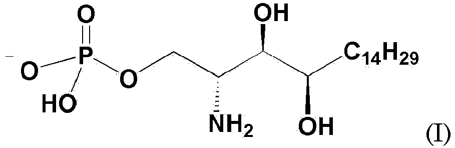

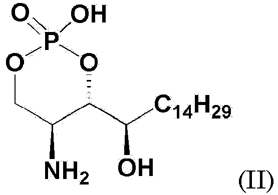

The compounds according to the present disclosure can be prepared using conventional knowledge known in the field of organic chemistry. For example, they may be prepared using the method disclosed in S. Li *et al.* (S. Li, WK Wilson, G. J. Schroepfer, Chemical synthesis of D-ribo-phytosphingosine-1-phosphate, potential modulator of cellular processes. J. Lipid Res. 40: 117-125, 1999) or using Korean Patent Publication No. 10-1514970.

A pharmaceutically acceptable salt of the compound of Chemical Formula I or II, or a solvate thereof can be appropriately prepared or selected by a person skilled in the art of organic chemistry using knowledge known in the art. The salt is physiologically acceptable and does not cause a typical allergic reaction or a reaction similar thereto when administered to humans, and the salt is preferably an acid addition salt formed by a free acid. The free acid may be an organic acid or an inorganic acid. The organic acid includes, but is not limited to, citric acid, acetic acid, lactic acid, tartaric acid, maleic acid, fumaric acid, formic acid, propionic acid, oxalic acid, trifluoroacetic acid, benzoic acid, gluconic acid, methanesulfonic acid, glycolic acid, succinic acid, 4-toluenesulfonic acid, glutamic acid, aspartic acid, and the like. In addition, the inorganic acid includes, but is not limited to, hydrochloric acid, hydrobromic acid, sulfuric acid, phosphoric acid, and the like. In one embodiment according to the present disclosure, the pharmaceutically acceptable salt may exist as an acid addition salt in which the compound of Chemical Formula I or II forms a salt together with a free acid. In addition, the compound of Chemical Formula I or II according to the present disclosure may include all salts, hydrates, and solvates that can be prepared by conventional methods as well as pharmaceutically acceptable salts. The compound of Chemical Formula I or II may be stabilized by an anion which may pair with an ammonium cation in the compound, and the anion may be any anion capable of pairing with an ammonium cation while being pharmaceutically acceptable. For example, the anion includes, but is not limited to, iodide (I⁻), sulfonate (SO₃²⁻), chloride (Cl⁻), and the like.

P1P and derivatives thereof according to the present disclosure can be effectively used in the treatment of sepsis or septic shock. The composition comprising P1P or a derivative thereof according to the present disclosure showed the effect of improving the survival rate when sepsis was induced by LPS injection or CLP surgery, which is representative methods of inducing sepsis, and P1P and its derivative drugs were treated. These drugs were confirmed to improve sepsis by inhibiting oxidative stress as well as inflammation. In addition, it was confirmed that P1P drugs can effectively treat sepsis at various stages, based on the result that P1P and derivatives thereof not only help to improve the proliferation and function of vascular endothelial cells, which are important for sepsis treatment, but also increase the amount of SIRT1 protein that is reduced in sepsis.

As used herein, the term "sepsis" refers to a systemic inflammatory response due to infection with bacteria or parasite, such as *Enterococcus sp., Staphylococcus sp., Streptococcus sp., Enterobacteriaceae* family, *Providencia sp.* and *Pseudomonas sp.,* and shows symptoms such as increased heart rate, hypotension, hypo or hyperthermia, rapid breathing, and increased or decreased white blood cell count.

As used herein, the term "treatment" refers to any action of inhibiting, eliminating, alleviating, ameliorating and/or improving a disease, or a symptom or condition caused by a disease, by administration of a composition.

As used herein, the term "prevention" refers to any action of inhibiting or delaying the onset of a disease or inhibiting or delaying the development of a symptom or condition due to a disease, by administration of a composition.

The composition of the present disclosure is prepared by including phytosphingosine-1-phosphate or a derivative thereof alone or in a mixture as an active ingredient. The content of phytosphingosine-1-phosphate or a derivative thereof included in the composition of the present disclosure is from about 0.005 to 0.5 % by weight, particularly from about 0.01 to 0.05 % by weight, based on the total weight of the total composition, but is not limited thereto.

The pharmaceutical composition according to the present disclosure may be administered simultaneously or sequentially, and the composition may be administered alone or in combination with other pharmaceutically active ingredients for the treatment of sepsis. In addition, the composition according to the present disclosure may comprise phytosphingosine-1-phosphate or a derivative thereof in combination of two or more.

The pharmaceutical composition comprising the active ingredient according to the present disclosure may further comprise suitable excipients such as carrier, diluent, preservative, stabilizer, wetting agent, emulsifier, solubilizer, sweetener, colorant, osmotic pressure regulator, antioxidant, *etc.* commonly used in the preparation of pharmaceutical compositions. Specifically, the excipients may include lactose, dextrose, sucrose, sorbitol, mannitol, xylitol, erythritol, maltitol, starch, acacia gum, alginate, gelatin, calcium phosphate, calcium silicate, cellulose, methylcellulose, microcrystalline cellulose, polyvinylpyrrolidone, water, methylhydroxybenzoate, propylhydroxybenzoate, talc, magnesium, stearate, mineral oil, *etc.*

The administration method of the pharmaceutical composition comprising the active ingredient according to the present disclosure can be easily selected according to the formulation, and can be administered by various routes. For example, the composition may be formulated in the form of powders, tablets, pills, granules, dragees, hard or soft capsules, liquids, emulsions, suspensions, syrups, elixirs, external preparations, suppositories, sterile injection solutions, *etc.* and may be administered orally or parenterally and systemically or locally.

Solid formulations for oral administration include tablets, pills, powders, granules, capsules, and the like, and such solid formulations are prepared by mixing the active ingredient of the present disclosure with at least one excipient, for example, starch, calcium carbonate, sucrose or lactose, gelatin, *etc.* In addition to simple excipients, lubricants such as magnesium stearate, talc are also used. Liquid formulations for oral administration include suspensions, internal liquids, emulsions, syrups, *etc.* In addition to water and liquid paraffin, which are commonly used simple diluents, various excipients such as wetting agents, sweeteners, fragrances, and preservatives may be included.

Formulations for parenteral administration include sterile aqueous solutions, non-aqueous solvents, suspensions, emulsions, freeze-dried preparations, suppositories, and nanoparticles (liposomes, nanoemulsions, micelles). Non-aqueous solvents and suspension solvents may include propylene glycol, polyethylene glycol, vegetable oils such as olive oil, and injectable esters such as ethyl oleate. As the base of the suppository, witepsol, macrogol, tween 61, cacao butter, laurin fat, glycerol, gelatin, *etc.* may be used. For administration in the form of nanoparticles, it may be administered in various forms such as liposomes, nanoemulsions, micelles, reverse micelles, and the like, and only examples using liposomes are shown herein, but the present disclosure is not limited to liposomes.

Furthermore, the pharmaceutical composition according to the present disclosure may be preferably formulated using appropriate methods known in the art or using methods disclosed in Remington's Pharmaceutical Science (recent edition), Mack Publishing Company, Easton PA.

The dosage of the pharmaceutical composition according to the present disclosure may vary depending on the patient's weight, age, sex, health status, diet, administration time, administration method, excretion rate and severity of disease, *etc.* An effective dosage for an adult (60 kg) is typically about 1-100 g/day, especially about 10-50 g/day, more preferably about 30 g/day. Since the dosage may vary depending on various conditions, it is apparent to those skilled in the art that the dosage may be adjusted, and therefore, the dosage is not intended to limit the scope of the present invention in any way.

The number of administration may be once a day or several times a day within a desired range, and the administration period is not particularly limited.

Hereinafter, examples are presented to help the understanding of the present invention. However, the following examples are only provided for easier understanding of the present invention, and the present invention is not limited to the following examples.

### Examples

### Experimental materials and method

### Preparation of P1P and derivatives thereof

Phytosphingosine-1-phosphate (P1P) and cP1P (O-cyclic P1P) were prepared as described in Korean Patent Publication No. 1514970. S1P and S1P agonist FTY720 (finolimod) were purchased from Sigma.

### Mouse model for sepsis

In order to develop a therapeutic agent for sepsis, methods for inducing sepsis in various ways have been developed. Typical methods used include administration of lipopolysacharide (LPS), which is a major causative agent for activating innate immune response, or cecal ligation and puncture (CLP) surgery. Direct administration of pathogenic microorganisms is also possible. Herein, LPS, which is an outer membrane component of Gram-negative bacteria called endotoxin, was administered or CLP surgery was performed.

### Experiments using LPS sepsis mouse model

For the experiment of the survival rate of mice after LPS administration, 7-week-old Balb/c mice were purchased from Orient Bio and adapted to the laboratory. Then, LPS (Sigma) was dissolved in phosphate buffer solution (PBS) at a concentration of 400 µg/mouse, 200 µg/mouse, 170 µg/mouse, 150 µg/mouse, 125 µg/mouse, and 100 µg/mouse, and 100 µl of each was injected into the abdominal cavity of a mouse. Thereafter, mouse survival rates were observed for 48 hours at 12 hour intervals.

### Measurement of the effect of P1P on sepsis

To this end, the LPS concentration was determined to be 150 µg/mouse, and the P1P drug was intraperitoneally injected at a concentration of 40 µg/mouse 5 and 60 minutes after intraperitoneal injection of LPS. The survival rate was measured by observation for 72 hours at 12 hour intervals. In another experiment, P1P drug was injected intraperitoneally at a concentration of 40 µg/mouse 1 hour after intraperitoneal injection of LPS of 125 µg/mouse to compare the survival rate of mice. In the same way, the survival rate was measured while changing the P1P concentration.

### Measurement of the effect of cP1P on sepsis

To this end, the LPS concentration was determined to be 150 µg/mouse, and the cP1P drug was intraperitoneally injected at a concentration of 40 µg/mouse 5 minutes after intraperitoneal injection of LPS. The survival rate was measured by observation for 72 hours at 12 hour intervals. In another experiment, cP1P drug was injected intraperitoneally at a concentration of 40 µg/mouse 1 hour after intraperitoneal injection of LPS of 125 µg/mouse to compare the survival rate of mice.

### Validation of drug efficacy on cytokine synthesis after LPS treatment in macrophages

The macrophage cell line used in the experiment was Raw 264.7 cell line purchased from ATCC, and Dulbeco's Modified Eagle's Media high glucose (with 10 % FBS, 0.5 % P/S) was used as a medium for culturing macrophages. Macrophages were cultured at 37 °C, 5 % CO₂ incubator. Macrophages were treated with P1P or cP1P drugs at concentrations of 0.02 µM, 0.1 µM, 0.5 µM and 1 µM, respectively and then LPS of 1 ng/ml after 6 hours, and cultured for 4 hours. And then changes of inflammatory cytokines (Tumor necrosis factor alpha, TNF-α) were measured by the manufacturer's method using ELISA (BD OptEIA^{™}). As a comparison group, S1P was used at the same concentration and conditions.

### Validation of drug efficacy on the proliferation of human vascular endothelial cells

Human umbilical vein endothelial cell (HUVEC) used in the experiment was purchased from Promocell, and cultured in an incubator at 37 °C, 5 % CO₂ using Endothelial Cell Growth Medium 2 as a culture medium. To confirm the proliferation effect of P1P drug, HUVEC cells were treated with P1P or cP1P drug at concentrations of 10 nM, 100 nM and 1000 nM, respectively, and cultured for 48 hours. The cell proliferation rate was measured according to the manufacturer's method using MTT assay (Molecular Probes). As a comparison group, S1P was used at the same concentration and conditions.

### Effects of drugs on apoptosis caused by oxidative stress in human vascular endothelial cells

Human umbilical vein endothelial cell (HUVEC) used in the experiment was purchased from Promocell, and cultured in an incubator at 37 °C, 5 % CO₂ using Endothelial Cell Growth Medium 2 as a culture medium. To confirm the efficacy of P1P drug in an oxidative stress environment, HUVEC cells were pretreated with P1P or cP1P at concentrations of 10 nM, 100 nM, and 1000 nM for 1 hour, and then exposed to oxidative stress by treatment with 500 µM hydrogen peroxide (H₂O₂) for 6 hours. The efficacy of the P1P drug on the survival of HUVEC cells after 6 hours of exposure to oxidative stress was confirmed by MTT assay. As a comparison group, S1P was used at the same concentration and conditions.

### Efficacy of P1P and cP1P drugs on expression of SIRT1 in macrophages

The macrophage cell line used in the experiment was Raw 264.7 cell line purchased from ATCC, and Dulbeco's Modified Eagle's Media high glucose (with 10 % FBS, 0.5 % P/S) was used as a medium for culturing macrophages. Macrophages were cultured in an incubator at 37 °C, 5 % CO₂. Macrophages were treated with P1P or cP1P drug at concentrations of 0.01 µM, 0.1 µM, and 1 µM, respectively and then LPS of 100 ng/ml after 4 hours, and cultured for 24 hours. The change in SIRT1 protein was measured by RT-PCR.

### Measurement of the effect of liposome nanoparticles to enhance the effect of P1P and cP1P drugs to treat sepsis

Since P1P and cP1P drugs are poorly soluble in water, a liposome formulation was prepared to improve the solubility and the efficacy of these drugs. Soybean lecithin called Lipoid S-75 was used to prepare the liposome, and 20 mg of lecithin was dissolved in 1 ml of chloroform. P1P or cP1P was dissolved in 1 ml of chloroform / methanol (2 : 1, v / v) solvent in an amount of 2 mg, 1 mg or 0.5 mg, respectively, and mixed with the lecithin solution. A thin lipid film was made by removing the organic solvent from the mixed solution using nitrogen gas or a rotary evaporator. The thin lipid film was stored in a vacuum desiccator overnight to completely remove the organic solvent remaining on the film. 5 ml of a phosphate buffer solution was added to the thin lipid film and stirred for 30 minutes to prepare multilamellar vesicles (MLV, large liposomes). The prepared MLV particles were sonicated for 2 minutes in an ice bath to make small unilamellar vesicles (SUV) of 100 nm or less. This sonication process was repeated twice. Only the supernatant obtained by centrifugation of the prepared SUV at 12 000 rpm for 5 minutes was used in the experiment. Liposomes containing P1P or cP1P drug (P1P-liposomes/cP1P-liposomes) were used in an experiment to increase the survival rate of mice using LPS. To this end, LPS of 170 µg/mouse, which is higher than the conventional LPS concentration (125-150 µg/mouse), was injected into the abdominal cavity of the mouse. P1P or cP1P drug was administered at 40 µg/mouse 5 minute after LPS administration, and 100 µl of P1P-liposomes and cP1P-liposomes (P1P and cP1P of about 40 µg/mouse) were injected into the abdominal cavity of the mouse, and the survival rate change was determined over time.

### Experiments using the CLP sepsis mouse model

Male C57BL/6 mice were used for CLP (cecal ligation and puncture) surgery, and 20 mice were used in each group (6-7 weeks of age and 18-20 g of body weight).

After the mice were anesthetized by inhalation with ether, sepsis was induced by CLP according to the method of Chaudry et al. (Surgery, 85(2), 205-211, 1979).

Specifically, to induce sepsis, a 2 cm incision was made in the abdomen to expose the cecum. A 5 mm area from the tip of the cecum was strongly tied with 3.0-silk suture and punctured with a 22-gauge needle to leak a small amount of excrement, and was put back into the abdominal cavity with the excrement, and the open area was stitched with 4.0-silk suture. For the negative control group (vehicle), the cecum was tied and exposed without perforation, and then was put back into the abdominal cavity and sutured.

6 and 18 hours after CLP treatment, cP1P-liposome (cP1P of 20 µg/mouse) was intravenously injected and the survival rate was observed for 120 hours.

### Example 1. Changes in mouse survival rate by LPS concentration

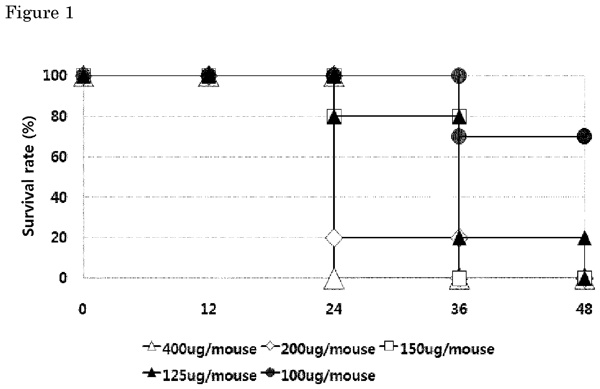

As described in the experimental method, the survival rate was observed at 12 hour intervals after intraperitoneal injection of LPS at various concentrations into Balb/c mice. In the case of 400 µg/mouse concentration, the survival rate dropped to 0 % before 24 hours, and at 200 µg/mouse concentration, the survival rate was about 20 % within 24 hours, and the survival rate fell to 0 % within 48 hours. When LPS was injected intraperitoneally at a concentration of 170 µg/mouse, the survival rate was 20 % within 24 hours, and the survival rate dropped to 0 % before 36 hours. At a concentration of 150-125 µg/mouse, the survival rate was about 80% within 24 hours, and the survival rate fell to 0 % within 36-48 hours. In the case of LPS concentration of 100 µg /mouse, a survival rate of 70 % or more was recorded (Table 1, Fig. 1). Therefore, the optimal LPS concentration for evaluating the efficacy of P1P and derivatives thereof was determined to be 150-125 µg/mouse. The concentration of LPS to measure the effect of the liposome nanoparticles containing the drug was determined to be 170 µg/mouse.

**Table 1. Changes in mouse survival rate by LPS concentration**

| Time (h) LPS conc. | Survival rate (%) | | | |
|---|---|---|---|---|
| | 0 h | 24 h | 36 h | 48 h |
| 400 µg/mouse | 100 % | 0 % | 0 % | 0 % |
| 200 µg/mouse | 100 % | 20 % | 0 % | 0 % |
| 170 µg/mouse | 100 % | 20 % | 0 % | 0 % |
| 150 µg/mouse | 100 % | 80 % | 0 % | 0 % |
| 125 µg/mouse | 100 % | 80 % | 20 % | 0 % |
| 100 µg/mouse | 100 % | 100 % | 70 % | 70 % |

### Example 2. Effects of P1P on changes in mouse survival rate by LPS treatment

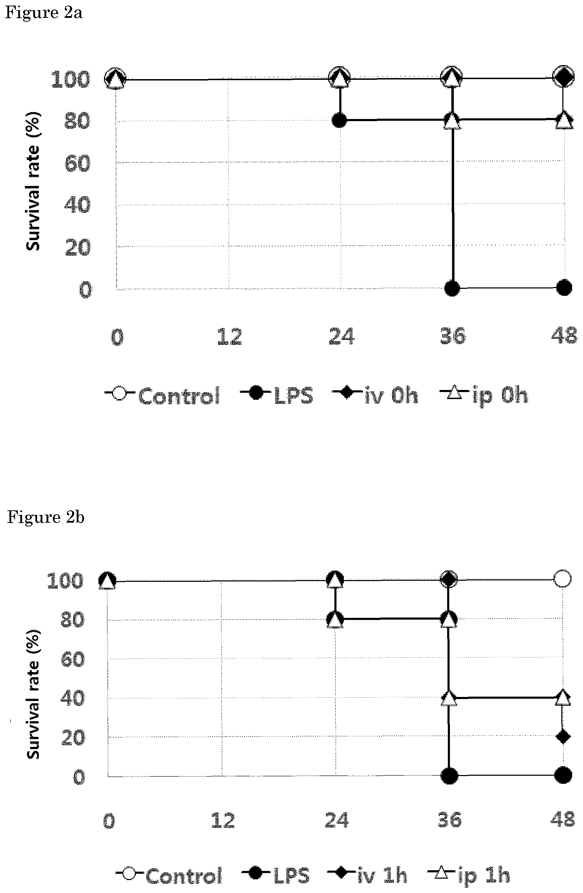

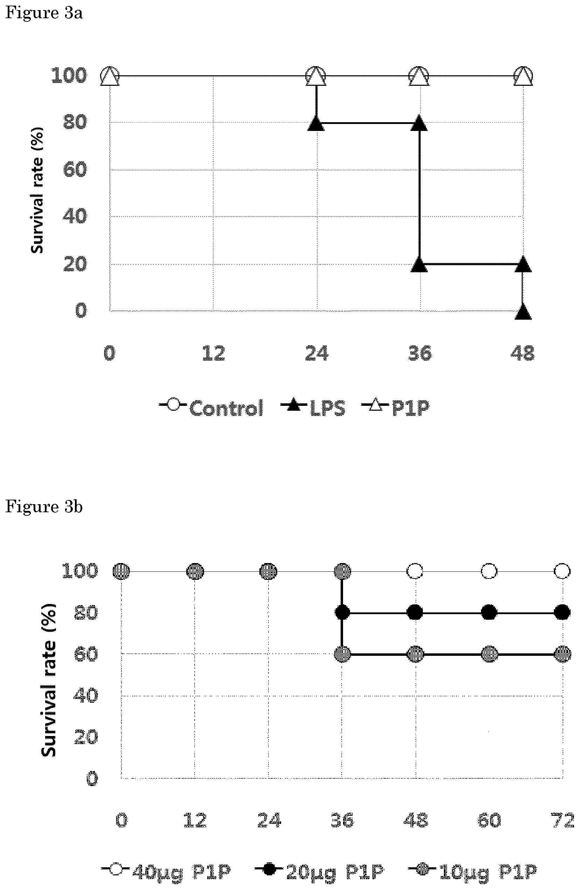

The effect of P1P to treat sepsis was tested under the same conditions as described in the experimental method. P1P of 40 µg/mouse was administered intraperitoneally and intravenously 5 minutes after intraperitoneal injection of LPS of 150 µg/mouse. In the control group not administered with the P1P drug, the survival rate dropped to 0 % within 36 hours (all fatal), but the groups in which the P1P was administered intraperitoneally and intravenously showed a survival rate of 80 % or more (Fig. 2a). When the P1P drug was administered 1 hour after LPS administration, the survival rate was about 40 % for intraperitoneal injection and 20 % for intravenous injection (Fig. 2b). These indicate that P1P effectively ameliorated sepsis induced after LPS treatment. Furthermore, P1P of 40 µg/mouse was administered by intraperitoneal injection 1 hour after intraperitoneal injection of 125 µg/mouse of LPS. In the control group not administered with the P1P drug, the survival rate dropped to 0 % within 48 hours, but the group administered with the intraperitoneal injection of P1P showed a survival rate of 100 % (Fig. 3a). In addition, as a result of observing the change in the survival rate of mice while varying the concentration of the P1P drug, all of them survived up to 72 hours at 40 µg/mouse concentration. In the case of 20 µg/mouse concentration, the survival rate was 80 %, and in the case of 10 µg/mouse concentration, the survival rate was about 60 % (Fig. 3b). It was confirmed that P1P increased the survival rate in a concentration-dependent manner.

### Example 3. Comparison of effects of S1P agonists and P1P on changes in mouse survival rate by LPS treatment

The therapeutic effects of S1P, S1P agonist (FTY720) and P1P for sepsis were compared under the same conditions as described in the experimental method. S1P, S1P agonist, and P1P drug were intraperitoneally injected at a concentration of 40 µg/mouse, respectively, 5 minutes after intraperitoneal injection of LPS at a concentration of 150 µg/mouse to compare the survival rates of mice. When LPS alone was administered, the survival rate was 80 % at 24 hours and 0 % after 36 hours, whereas when FTY720 (fingolimod) was administered, the survival rate was 40 % at 24 hours and 20 % after 36 hours (Table 2). In the case of FTY720, the survival rate within 24 hours was lower than that of the LPS-treated group. In the case of S1P treatment, the survival rate was 60 % or more within 24 hours, and was 40 % at 36 hours, and 40 % was maintained until the end of the experiment. On the other hand, in the case of P1P drug treatment, 100 % survival rate was shown within 24 hours, and the survival rate was 80 % or more from 36 hours to the end of the experiment. These results show that the P1P drug has a much higher therapeutic effect than FTY720, which is an S1P agonist used to develop therapeutic agents for sepsis, and has greater therapeutic effect on sepsis than S1P. This is also in good agreement with the anti-inflammatory effect.

**Table 2. Comparison of therapeutic effects of S1P agonists and P1P in LPS-induced sepsis model**

| Group Time (h) | Survival rate (%) | | | | |
|---|---|---|---|---|---|
| | Control | LPS | LPS+S1P | LPS+FTY720 | LPS+P1P |
| 0 | 100 | 100 | 100 | 100 | 100 |
| 12 | 100 | 100 | 100 | 100 | 100 |
| 24 | 100 | 80 | 60 | 40 | 100 |
| 36 | 100 | 0 | 40 | 20 | 80 |
| 48 | 100 | 0 | 40 | 20 | 80 |
| 72 | 100 | 0 | 40 | 20 | 80 |

### Example 4. Effects of cP1P on changes in mouse survival rate by LPS treatment

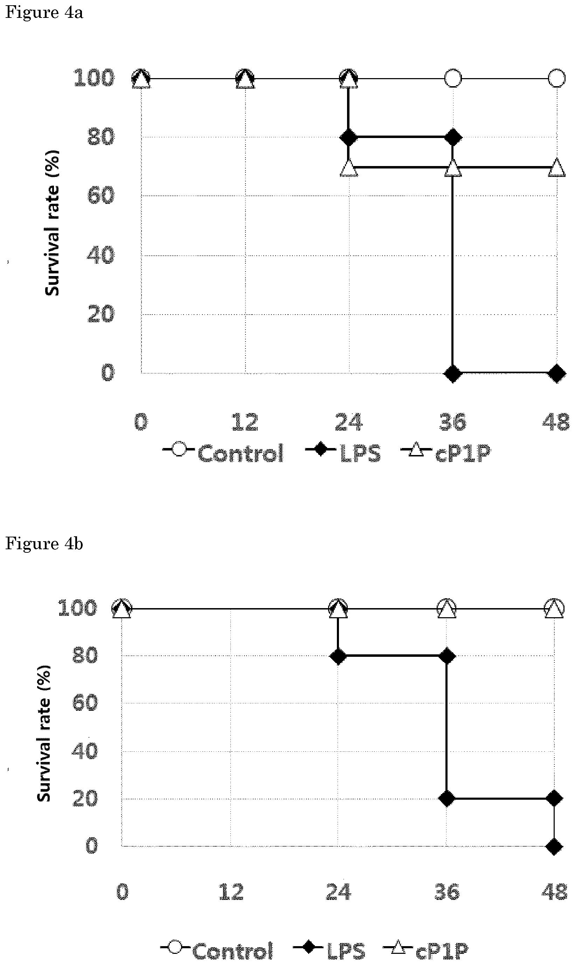

The therapeutic effect of cP1P for sepsis was tested under the same conditions as described in the experimental method. cP1P of 40 µg/mouse was intraperitoneally administered 5 minutes after intraperitoneal injection of LPS of 150 µg/mouse. In the control group not administered with the cP1P drug, the survival rate dropped to 0 % within 36 hours, but the group treated with cP1P showed a survival rate of 70 % or more (Fig. 4a). When the cP1P drug was administered 1 hour after intraperitoneal injection of LPS of 125 µg/mouse, the survival rate was about 100 %, but the LPS-treated group showed 0 % survival rate at 48 hours (Fig. 4b). These results indicate that cP1P can effectively ameliorate sepsis induced by LPS treatment.

### Example 5. Efficacy of P1P and derivatives thereof on the secretion of inflammatory cytokines in macrophage cell lines

Since sepsis leads to an excessive cytokine storm following the initial infection, inhibition of cytokine secretion can be an effective criterion for judging the effectiveness of treatment.

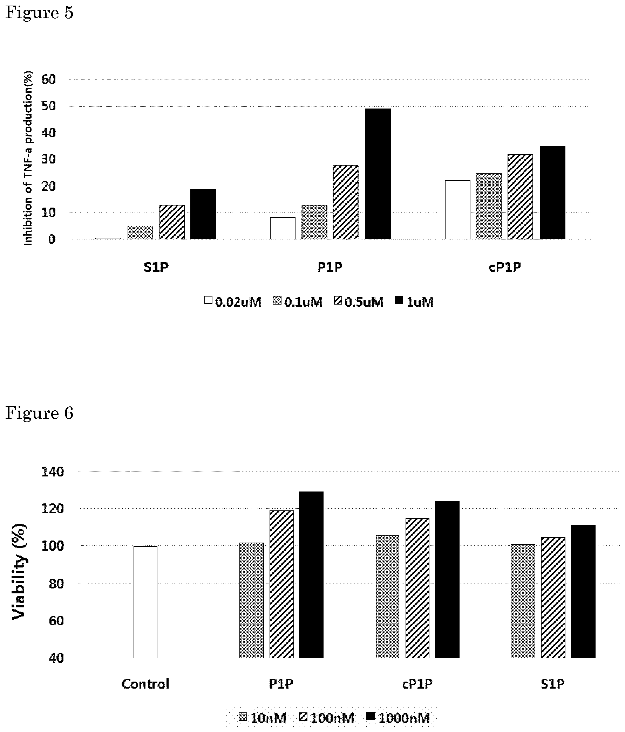

To this end, macrophage cell lines were treated with LPS to increase inflammatory cytokines, and the efficacy of P1P and derivatives thereof on inflammatory cytokine secretion was evaluated. Macrophage cell lines were treated with P1P drug or cP1P drug, and 6 hours later, treated with LPS at a concentration of 500 ng/ml for 4 hours. As a result, it was confirmed by the ELISA method that inflammatory cytokines (tumor necrosis factor alpha, TNF-α) were significantly increased in the LPS-treated group than in the control group, and the secretion of inflammatory cytokines was reduced in the group treated with the P1P drug or the cP1P drug. P1P (49 % inhibitory efficacy at 1 µM concentration) and cP1P (35 % inhibitory efficacy at 1 µM concentration) had significantly higher inhibitory efficacy on inflammatory cytokine production than S1P (19 % inhibitory efficacy at 1 µM concentration) used as a positive control (Fig. 5). This proves the superior effect of the present pharmaceutical composition.

### Example 6. Effects of P1P and cP1P drugs on the proliferation of human vascular endothelial cells

The activity and proliferation of vascular endothelial cells is very important in the treatment of sepsis. Therefore, the proliferation of vascular endothelial cells was determined after treatment with the P1P drug or cP1P drug, and it was found that the proliferation occurred better as the concentrations of the P1P drug and cP1P drug increased, and the efficacy on the proliferation was higher than that of S1P used as a positive control (Fig. 6).

### Example 7. Efficacy of P1P and cP1P drugs on survival of vascular endothelial cells by oxidative stress

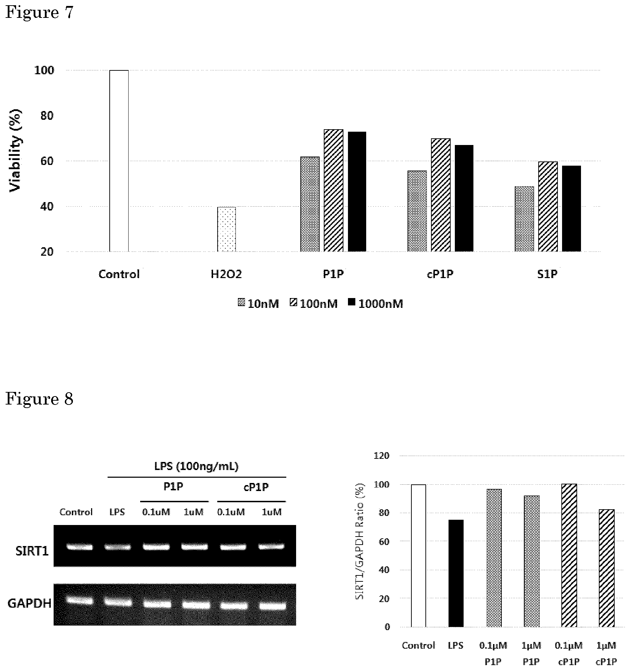

In addition to causing inflammation, LPS promotes oxidative stress to promote vascular endothelial cell death. To analyze the effect of the present drugs, oxidative stress was induced in vascular endothelial cells by treatment with 500 µM of hydrogen peroxide. As a result, when the P1P drug was treated, the cell viability was 70 % or more, while the control group not treated with the drug showed a cell viability of about 40 %. Further, P1P and cP1P showed higher cell viability than S1P used as a control group (Fig. 7)

### Example 8. Efficacy of P1P and cP1P drugs on changes in SIRT1 protein

Expression of SIRT1 protein is very important in the treatment of sepsis. When sepsis is induced, the synthesis and activity of SIRT1 protein is reduced. Macrophages were treated with P1P drugs during culture, and the expression level of SIRT1 protein was confirmed by RT-PCR 24 hours later. As a result, it was found that treatment with P1P and cP1P increased SIRT1 protein even at very low concentrations (Fig. 8).

### Example 9. Effects of liposomal P1P and liposomal cP1P on the survival rate of mice by LPS treatment (Efficacy of liposomal nanoparticles to increase the therapeutic effect of P1P and cP1P drugs for sepsis)

In order to improve the solubility and efficacy of P1P and cP1P, liposome nanoparticles were used and tested under the same conditions as described in the experimental method. In the control group treated only with LPS of 170 µg/mouse, the survival rate was about 20 % within 24 hours, and the survival rate dropped to 0 % within 36 hours. In the group treated with only the P1P drug after LPS treatment, the survival rate was 40 % after 24 hours and 20 % after 36 hours. However, when 40 µg of P1P was formulated into liposome nanoparticles and administered, the survival rate was 80 % from 24 hours to the end of the experiment (Table 3).

In the same way, when the cP1P drug was applied, when only 40 µg/mouse of the cP1P drug was administered after LPS treatment, the survival rate was 60 % for 24 hours, and the survival rate was 40 % from 36 hours to the end of the experiment. Like the P1P drug, when cP1P drug was formulated into liposome nanoparticles and then administered, the survival rate was 100 % by 24 hours, and the survival rate was 80 % from 36 hours to the end of the experiment. It can be seen that when P1P and cP1P drugs are formulated into liposome nanoparticles and administered, not only solubility is improved, but also therapeutic effect is significantly increased (Table 3).

**Table 3. Effects of liposome carriers on enhancing the therapeutic effect for LPS-induced sepsis**

| Group | Survival rate (%) | | | | | |
|---|---|---|---|---|---|---|
| Time (h) | Control | LPS | LPS+P1P | LPS+cP1P | LPS+P1P liposome | LPS+cP1P liposome |
| 0 | 100 | 100 | 100 | 100 | 100 | 100 |
| 12 | 100 | 80 | 80 | 80 | 100 | 100 |
| 24 | 100 | 20 | 40 | 60 | 80 | 100 |
| 36 | 100 | 0 | 20 | 40 | 80 | 80 |
| 48 | 100 | 0 | 20 | 40 | 80 | 80 |
| 72 | 100 | 0 | 20 | 40 | 80 | 80 |

### Example 10. Comparison of therapeutic effects according to the concentration of liposomal P1P and liposomal cP1P on the survival rate of mice by LPS treatment

Since the therapeutic effect of P1P and cP1P drugs was improved through liposome formulation in Example 9, the therapeutic effects according to the concentrations of P1P and cP1P drugs in liposome nanoparticles were compared. Survival rates were compared while fixing the concentration of the phospholipid constituting the liposome and varying the concentration of the drug. For the P1P drug, concentrations of 40, 20, and 10 µg were used, and the phospholipid constituting the liposome was administered at 400 µg. When LPS was administered at a concentration of 170 µg/mouse and then the P1P drug was administered at a concentration of 40µg/mouse, the survival rate was 80 % at 12 hours, but decreased to 40 % at 24 hours and was 20 % after 36 hours. However, when administered as liposome nanoparticles, the survival rate increased to about 80 %, and even when the concentration of the P1P drug was 20 µg/mouse, the survival rate was 80 %. And even when the concentration of the P1P drug was 10 µg/mouse, the survival rate was 60 % or more. As described above, when the P1P drug is formulated into liposome nanoparticles, even if the concentration of the drug is lowered, an excellent therapeutic effect is shown (Table 4). Although the cP1P drug showed similar effects to the P1P drug, when only the cP1P drug was administered, a 40 % survival rate was shown, whereas when administered as liposome nanoparticles, a survival rate of 80 % or more was shown even at a concentration of 10 µg/mouse (Table 5). As described above, it can be seen that the use of liposome nanoparticles not only enhances the therapeutic effect, but also provides the same therapeutic effect even when the concentration of the drug is lowered.

**Table 4. Comparison of effects of liposomal P1P concentration to increase therapeutic effect for sepsis by LPS administration**

| Group | Survival rate (%) | | | | | |
|---|---|---|---|---|---|---|
| Time (h) | Control | LPS | LPS+P1P (40 µg) | LPS+liposomal P1P (40 µg) | LPS+ liposomal P1P (20 µg) | LPS+ liposomal P1P (10 µg) |
| 0 | 100 | 100 | 100 | 100 | 100 | 100 |
| 12 | 100 | 80 | 80 | 100 | 100 | 80 |
| 24 | 100 | 20 | 40 | 80 | 80 | 60 |
| 36 | 100 | 0 | 20 | 80 | 80 | 60 |
| 48 | 100 | 0 | 20 | 80 | 80 | 60 |
| 72 | 100 | 0 | 20 | 80 | 80 | 60 |

**Table 5. Comparison of effects of liposomal cP1P concentration to increase therapeutic effect for sepsis by LPS administration**

| Group | Survival rate (%) | | | | | |
|---|---|---|---|---|---|---|
| Time (h) | Control | LPS | LPS+cP1P (40 µg) | LPS+liposomal cP1P (20 µg) | LPS+ liposomal cP1P (10 µg) | LPS+ liposomal cP1P (5 µg) |
| 0 | 100 | 100 | 100 | 100 | 100 | 100 |
| 12 | 100 | 80 | 80 | 100 | 100 | 100 |
| 24 | 100 | 20 | 60 | 100 | 100 | 60 |
| 36 | 100 | 0 | 40 | 80 | 80 | 60 |
| 48 | 100 | 0 | 40 | 80 | 80 | 60 |
| 72 | 100 | 0 | 40 | 80 | 80 | 60 |

### Example 11. Effects of liposomal cP1P on mouse survival rate by CLP treatment

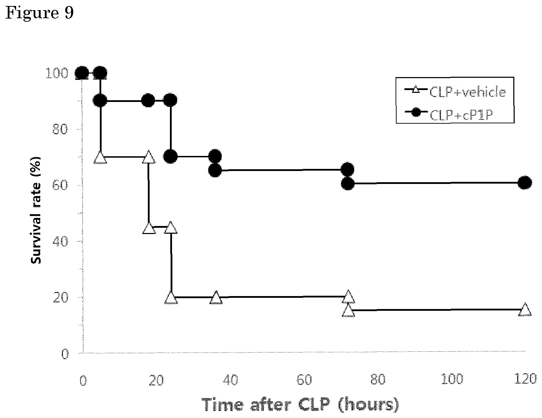

In order to confirm the therapeutic effect of cP1P for sepsis after CLP treatment, cP1P liposome nanoparticles were used and tested under the same conditions as described in the experimental method. As a result, in the case of the negative control group, the survival rate was about 15 %, but the survival rate for the group administered with cP1P-liposome twice at 6 hours and 18 hours was 60 % or more (Fig. 9). When cP1P-liposome was administered intravenously 6 hours after CLP treatment, the survival rate of mice was improved. This indicates a high potential for use as a therapeutic agent for sepsis.

Taken together, the pharmaceutical composition comprising P1P or a derivative thereof according to the present disclosure not only improves the survival rate of sepsis mice induced by LPS treatment and CLP treatment, but also reduces cytokine secretion, and enhances proliferation and function of vascular endothelial cells. Further, the composition increases the expression of SIRT1 protein, which is decreased in sepsis patients, and shows an excellent therapeutic effect for sepsis compared to the S1P. In addition, the solubility and therapeutic efficacy of P1P and cP1P drugs are improved when formulated into liposome nanoparticles.

Although the exemplary embodiments of the present disclosure have been described in detail above, the scope of the present disclosure is not limited thereto, and various modifications and improvements by those skilled in the art using the basic concept of the present disclosure as defined in the following claims are also included in the scope of the present disclosure.

All technical terms used in the present disclosure, unless otherwise defined, have the same meaning as commonly understood by one of ordinary skill in the art of the present disclosure. The contents of all the publications described herein are incorporated herein by reference.

## Claims

1. A pharmaceutical composition for preventing or treating sepsis or septic shock comprising phytosphingosine-1-phosphate (P1P), cP1P (O-cyclic P1P), or a pharmaceutically acceptable salt thereof.

2. The pharmaceutical composition for preventing or treating sepsis or septic shock according to claim 1, wherein the sepsis or septic shock is induced by Gram-negative bacteria.

3. The pharmaceutical composition for preventing or treating sepsis or septic shock according to claim 1, wherein the sepsis or septic shock is induced by Gram-negative bacteria-derived lipopolysaccharide (LPS).

4. The pharmaceutical composition for preventing or treating sepsis or septic shock according to claim 1, wherein the pharmaceutical composition reduces the secretion of inflammatory cytokines.

5. The pharmaceutical composition for preventing or treating sepsis or septic shock according to claim 4, wherein the cytokine is TNF-α.

6. The pharmaceutical composition for preventing or treating sepsis or septic shock according to claim 1, wherein the pharmaceutical composition promotes proliferation of vascular endothelial cells.

7. The pharmaceutical composition for preventing or treating sepsis or septic shock according to claim 1, wherein the pharmaceutical composition is a nanoparticle formulation selected from the group consisting of liposomes, nanoemulsions and micelles.

8. A method for preventing or treating sepsis or septic shock comprising administering to a subject in need thereof an effective amount of phytosphingosine-1-phosphate (P1P), cP1P (O-cyclic P1P), or a pharmaceutically acceptable salt thereof.

9. Use of phytosphingosine-1-phosphate (P1P), cP1P (O-cyclic P1P) or a pharmaceutically acceptable salt thereof for preventing or treating sepsis or septic shock.
